# EUROPEAN PATENT APPLICATION

(11) **EP 4 566 588 A1**
(43) Date of publication of application: **11.06.2025**
(21) Application number: 23848135.2
(22) Date of filing: 05.07.2023
(51) Int. Cl.: A61K 9/107, A61K 38/13, A61K 47/10, A61K 47/24, A61K 47/14, A61P 27/02, A61P 37/06

(54) **CYCLOSPORIN EMULSION AND METHOD FOR PREPARING SAME**

(30) Priority: 03.08.2022 CN 202210929871
(71) Applicant: Zhuhai Essex Bio-pharmaceutical Co., Ltd., Zhuhai, Guangdong 519088 (CN)
(72) Inventor: ZHANG, Shurong, Zhuhai, Guangdong 519088 (CN); NGIAM, Malcolm, Zhuhai, Guangdong 519088 (CN); XUE, Qi, Zhuhai, Guangdong 519088 (CN); NIU, Lu, Zhuhai, Guangdong 519088 (CN); WANG, Zhenheng, Zhuhai, Guangdong 519088 (CN); XIONG, Yingluo, Zhuhai, Guangdong 519088 (CN); DAI, Peimin, Zhuhai, Guangdong 519088 (CN); WANG, Yunzhe, Zhuhai, Guangdong 519088 (CN)
(74) Representative: Patentanwälte Ruff, Wilhelm, Beier, Dauster & Partner mbB
(86) International application number: PCT/CN2023/105857
(87) International publication number: WO 2024/027451

(57) **Abstract**

The present invention relates to a cyclosporine emulsion and preparation method thereof, in particular to an oil-in-water cyclosporine emulsion, comprising an interfacial stabilizer, wherein the interfacial stabilizer comprises a combination of polyethylene glycol 15-hydroxystearate and a phospholipid. The emulsion has long-term stability in terms of the pH value, particle size and encapsulation rate.

## Description

### Technical Field

The present invention relates to the field of formulations, in particular to a cyclosporine emulsion and a preparation method thereof.

### Background Art

Cyclosporine is a calcineurin inhibitor and an effective immunosuppressant. Compared with other immunosuppressants, it has less toxic effects and can selectively change the function of T lymphocytes in a reversible manner, prevent the transcription of lymphokine genes, interfere with the transmission of information, and inhibit the release of interleukin-2 (II,- 2), interferon and other immune factors, thereby exerting anti-inflammatory effects. After ocular administration, cyclosporine can induce the inactivation of calcineurin, and prevent the release of pro-inflammatory cytokines such as IL-2, thereby alleviating the symptoms of dry eyes (keratoconjunctivitis sicca).

Cyclosporine is almost insoluble in water, but has good lipid solubility. For commercially available eye drops, it is generally dissolved in vegetable oil to make a solution or made into an oil-in-water emulsion using emulsification technology. Oil solutions are highly irritating when used for local administration to the eye, and patient compliance is poor. In the field of oil-in-water ophthalmic emulsions, the marketed Restasis^{®} and Ikervis^{®} are both submicron emulsions with an average particle size of 100 nm or above, and are opaque milky white in nature. After eye drops, they will blur the patient's vision. In addition, Restasis^{®} has a low bioavailability due to its large particle size and the lack of components that promote drug retention or penetration in the eye; Ikervis^{®} uses the cationic surfactant, cetalkonium chloride to enhance the retention time of the drug on the ocular surface, but the use of cationic surfactants increases the occurrence of adverse reactions such as eye pain, eye irritation, and eye congestion.

A nanoemulsion is a translucent to almost transparent dispersion system with particle size below 100 nm formed by self-assembly of oil, water, interfacial stabilizers, etc. Nanoemulsion is used for ocular drug delivery. Its nano-sized particle size can increase the permeability of drugs in ocular tissues and improve drug efficacy. Higher clarity can improve patient compliance. It has high development value.

At present, oil-in-water ophthalmic emulsions need to solve two major technical problems: 1) emulsion particle stability and encapsulation stability problems, for example, the ionic strength of physiological tears or artificial tear substitutes will affect the stability of the interfacial film of the emulsion, which will lead to emulsion particle aggregation and drug leakage, affecting the safety and effectiveness of the formulation; 2) during long-term storage, the pH value of the formulation will gradually decrease, affecting the quality of the formulation. Oil-in-water emulsions usually use sodium hydroxide or hydrochloric acid to adjust the pH value, but during storage, as the oil lipid is hydrolyzed into free fatty acids, the pH value will gradually decrease. The use of buffer salts to form a buffer system can improve the stability of the pH value, but the increase in ionic strength will affect the stability of the interfacial film, which will lead to emulsion particle aggregation and drug leakage. In view of this, in the field of oil-in-water ophthalmic emulsions, it is urgent to provide a stabilizer combination that can produce a synergistic stabilizing effect to jointly form an interfacial composite film. The composite film can remain stable in a high ionic strength environment (such as a buffered salt solution), thereby achieving long-term stability of the pH value, particle size and encapsulation rate of the formulation.

### Summary of the invention

The object of the present invention is to provide a cyclosporine emulsion with a controllable particle size (for example, an average particle size below 50 nm) and a high encapsulation rate (above 90%).

Another object of the present invention is to provide a cyclosporine emulsion with long-term stable pH value, particle size and encapsulation rate.

The present invention provides an oil-in-water cyclosporine emulsion, comprising an interfacial stabilizer, wherein the interfacial stabilizer comprises a combination of polyethylene glycol 15-hydroxystearate and phospholipid.

The present invention also provides a method for preparing an oil-in-water cyclosporine emulsion, wherein the emulsion comprises an interfacial stabilizer, an osmotic pressure regulator and a buffer salt, wherein the interfacial stabilizer comprises a combination of polyethylene glycol 15-hydroxystearate and a phospholipid, and the method comprises
1) mixing the oil and the interfacial stabilizer uniformly, adding cyclosporine thereto and dissolving the cyclosporine to form an oil phase;
2) dissolving a portion of the osmotic pressure regulator in water, and stirring and mixing the oil phase and the water containing the osmotic pressure regulator to form a primary emulsion; and
3) dissolving the buffer salt and the remaining osmotic pressure regulator in water to form a solution, and adding the primary emulsion to the solution and mixing well.

The cyclosporine emulsion has improved interfacial film stability, can effectively inhibit the migration of cyclosporine into the aqueous medium, and inhibit the aggregation of emulsion particles, so that the long-term stability in terms of pH value, particle size and encapsulation rate is significantly improved. Due to the permeability promotion and higher clarity of the nano-sized particles, the emulsion has higher efficacy and better patient compliance.

### DETAILED DESCRIPTION

Unless otherwise defined, all technical and scientific terms used herein have the same meanings as are commonly understood by those skilled in the art. In the event of a conflict, the present document, including definitions, shall control. Preferred methods and materials are described below, but methods and materials similar or equivalent to those described herein can be used to carry out or test the present invention. The materials, methods, and examples disclosed herein are illustrative only and are not intended to be limiting.

For all numerical ranges involved in the present disclosure, it should be understood that all specific values within the range are disclosed, as well as sub-ranges defined by any two values within the range. For example, for 1-20, it should be understood that specific values such as 1, 2, 3, 3.5, 4.5, 10, 12, 15, 20, and sub-ranges such as 1-5, 2-6, 3.5-7.5, 15-20 are disclosed.

The present invention relates to an oil-in-water cyclosporine emulsion, preferably a nanoemulsion, comprising an interfacial stabilizer, wherein the interfacial stabilizer comprises a combination of polyethylene glycol 15-hydroxystearate and phospholipid.

In one embodiment, the oil-in-water cyclosporine emulsion further comprises a buffer salt.

In one embodiment, the phospholipid is selected from the group consisting of soybean lecithin, egg yolk lecithin, and any combination thereof, preferably, selected from the group consisting of soybean lecithin S100, egg yolk lecithin E80, and any combination thereof.

In one embodiment, the mass ratio of cyclosporine to polyethylene glycol 15-hydroxystearate is 1:30 to 1:45.

In one embodiment, the mass ratio of phospholipid to polyethylene glycol 15-hydroxystearate is 1:14 to 1:31.

In one embodiment, the content of polyethylene glycol 15-hydroxystearate is 2.25-13.5 g/100 ml.

In one embodiment, the buffer salt is a phosphate buffer salt, and preferably, the buffer salt concentration is 0.01 mol/L to 0.05 mol/L calculated on the basis of phosphate.

In one embodiment, the oil-in-water cyclosporine emulsion further comprises an osmotic pressure regulator; preferably, the osmotic pressure regulator is selected from the group consisting of sodium chloride, sorbitol, and any combination thereof; preferably, the content of the osmotic pressure regulator is 0.2-1 g/100 ml.

In one embodiment, the average particle size of the particles of the emulsion does not exceed 50 nm, and preferably is 10-40 nm, more preferably 20-30 nm. Preferably, 90% of the particles of the emulsion have a particle size of no more than 90 nm. Preferably, the pH value of the emulsion is 6.5-8.0, preferably 6.8-7.6, more preferably 7.0-7.4. Preferably, the encapsulation rate of the emulsion is not less than 90%, preferably not less than 95%. Preferably, the emulsion is a transparent milky liquid. Preferably, the emulsion is an eye drop. Preferably, the oil is selected from the group consisting of medium-chain triglycerides, soybean oil, castor oil, olive oil, and fish oil.

The present invention also provides a method for preparing an oil-in-water cyclosporine emulsion, wherein the emulsion comprises an interfacial stabilizer, an osmotic pressure regulator and a buffer salt, wherein the interfacial stabilizer comprises a combination of polyethylene glycol 15-hydroxystearate and a phospholipid, and the method comprises
1) mixing the oil and the interfacial stabilizer uniformly, adding cyclosporine thereto and dissolving the cyclosporine to form an oil phase;
2) dissolving a portion of the osmotic pressure regulator in water, and stirring and mixing the oil phase and the water containing the osmotic pressure regulator to form a primary emulsion; and
3) dissolving the buffer salt and the remaining osmotic pressure regulator in water to form a solution, and adding the primary emulsion to the solution and mixing well.

The buffer salt is preferably phosphate buffer salt.

The technical features in the various embodiments mentioned above can be combined with each other to form another technical solution without causing any contradiction.

### Example

### Example 1

Cyclosporine nanoemulsion was prepared using polyethylene glycol 15-hydroxystearate and soybean lecithin S100 as stabilizers, disodium hydrogen phosphate and sodium dihydrogen phosphate as pH regulators, and sodium chloride as an osmotic pressure regulator. The formulation composition is as follows:

| **Composition** | **Function** | **Amount** |
|---|---|---|
| cyclosporine | active substance | 0.1 g |
| oil (medium chain triglycerides) | dispersed phase | 1.5 g |
| polyethylene glycol 15-hydroxystearate | stabilizer | 4.5 g |
| soybean lecithin S100 | stabilizer | 0.273 g |
| sodium chloride | osmotic pressure regulator | 0.356 g |
| disodium hydrogen phosphate (anhydrous basis) | pH regulator | 0.511 g |
| sodium dihydrogen phosphate (anhydrous basis) | pH regulator | 0.168 g |
| water for injection | dispersing agent | up to 100 ml |

| **Mass ratio/concentration** | | |
|---|---|---|
| mass ratio of cyclosporine to polyethylene glycol 15-hydroxystearate | 1:45 | |
| mass ratio of phospholipid to polyethylene glycol 15-hydroxystearate | 1:16 | |
| phosphate concentration | 0.05 mol/L | |

### Preparation method:

1) polyethylene glycol 15-hydroxystearate, soybean lecithin S100 and oil were added into container 1, heated to 60 °C - 80 °C, and stirred to mix well; and cyclosporine was added to dissolve with stirring to form an oil phase;
2) about 9% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 50% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Example 2

Cyclosporine nanoemulsion was prepared using polyethylene glycol 15-hydroxystearate and egg yolk lecithin E80 as stabilizers, disodium hydrogen phosphate and sodium dihydrogen phosphate as pH regulators, and sodium chloride as an osmotic pressure regulator. The formulation composition is as follows:

| **Composition** | **Function** | **Amount** |
|---|---|---|
| cyclosporine | active substance | 0.1 g |
| oil (medium chain triglycerides) | dispersed phase | 1.5 g |
| polyethylene glycol 15-hydroxystearate | stabilizer | 4.5 g |
| egg yolk lecithin E80 | stabilizer | 0.273 g |
| sodium chloride | osmotic pressure regulator | 0.356 g |
| disodium hydrogen phosphate (anhydrous basis) | pH regulator | 0.511 g |
| sodium dihydrogen phosphate (anhydrous basis) | pH regulator | 0.168 g |
| water for injection | dispersing agent | up to 100ml |

| **Mass ratio/concentration** | | |
|---|---|---|
| mass ratio of cyclosporine to polyethylene glycol 15-hydroxystearate | 1:45 | |
| mass ratio of phospholipid to polyethylene glycol 15-hydroxystearate | 1:16 | |
| phosphate concentration | 0.05 mol/L | |

### Preparation method:

1) polyethylene glycol 15-hydroxystearate, egg yolk lecithin E80 and oil were added into container 1, heated to 60 °C - 80 °C, and stirred to mix well; and cyclosporine was added to dissolve with stirring to form an oil phase;
2) about 9% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 50% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Example 3

Cyclosporine nanoemulsion was prepared using polyethylene glycol 15-hydroxystearate and soybean lecithin S100 as stabilizers, disodium hydrogen phosphate and sodium dihydrogen phosphate as pH regulators, and sodium chloride and sorbitol as osmotic pressure regulators. The formulation composition is as follows:

| **Composition** | **Function** | **Amount** |
|---|---|---|
| cyclosporine | active substance | 0.1 g |
| oil (medium chain triglycerides) | dispersed phase | 1.5 g |
| polyethylene glycol 15-hydroxystearate | stabilizer | 4.5 g |
| soybean lecithin S100 | stabilizer | 0.273 g |
| sodium chloride | osmotic pressure regulator | 0.306 g |
| sorbitol | osmotic pressure regulator | 0.675 g |
| disodium hydrogen phosphate (anhydrous basis) | pH regulator | 0.547 g |
| sodium dihydrogen phosphate (anhydrous basis) | pH regulator | 0.137 g |
| water for injection | dispersing agent | up to 100ml |

| **Mass ratio/concentration** | | |
|---|---|---|
| mass ratio of cyclosporine to polyethylene glycol 15-hydroxystearate | | 1:45 |
| mass ratio of phospholipid to polyethylene glycol 15-hydroxystearate | | 1:16 |
| Phosphate concentration | | 0.05 mol/L |

### Preparation method:

1) polyethylene glycol 15-hydroxystearate, soybean lecithin S100 and oil were added into container 1, heated to 60 °C - 80 °C, and stirred to mix well; and cyclosporine was added to dissolve with stirring to form an oil phase;
2) about 9% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 50% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Example 4

Cyclosporine nanoemulsion was prepared using polyethylene glycol 15-hydroxystearate and soybean lecithin S100 as stabilizers, disodium hydrogen phosphate and sodium dihydrogen phosphate as pH regulators, and sodium chloride as an osmotic pressure regulator. The formulation composition is as follows:

| **Composition** | **Function** | **Amount** |
|---|---|---|
| cyclosporine | active substance | 0.3 g |
| oil (medium chain triglycerides) | dispersed phase | 4.5 g |
| polyethylene glycol 15-hydroxystearate | stabilizer | 13.5 g |
| soybean lecithin S100 | stabilizer | 0.819 g |
| sodium chloride | osmotic pressure regulator | 0.23 g |
| disodium hydrogen phosphate (anhydrous basis) | pH regulator | 0.102 g |
| sodium dihydrogen phosphate (anhydrous basis) | pH regulator | 0.034 g |
| water for injection | dispersing agent | up to 100ml |

| **Mass ratio/concentration** | | |
|---|---|---|
| mass ratio of cyclosporine to polyethylene glycol 15-hydroxystearate | | 1:45 |
| mass ratio of phospholipid to polyethylene glycol 15-hydroxystearate | | 1:16 |
| phosphate concentration | | 0.01 mol/L |

### Preparation method:

1) polyethylene glycol 15-hydroxystearate, soybean lecithin S100 and oil were added into container 1, heated to 60 °C - 80 °C, and stirred to mix well; and cyclosporine was added to dissolve with stirring to form an oil phase;
2) about 27% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 30% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Example 5

Cyclosporine nanoemulsion was prepared using polyethylene glycol 15-hydroxystearate and soybean lecithin S100 as stabilizers, disodium hydrogen phosphate and sodium dihydrogen phosphate as pH regulators, and sodium chloride as an osmotic pressure regulator. The formulation composition is as follows:

| **Composition** | **Function** | **Amount** |
|---|---|---|
| cyclosporine | active substance | 0.05 g |
| oil (medium chain triglycerides) | dispersed phase | 0.75 g |
| polyethylene glycol 15-hydroxystearate | stabilizer | 2.25 g |
| soybean lecithin S100 | stabilizer | 0.137 g |
| sodium chloride | osmotic pressure regulator | 0.628 g |
| disodium hydrogen phosphate (anhydrous basis) | pH regulator | 0.256 g |
| sodium dihydrogen phosphate (anhydrous basis) | pH regulator | 0.084 g |
| water for injection | dispersing agent | up to 100ml |

| **Mass ratio/concentration** | | |
|---|---|---|
| mass ratio of cyclosporine to polyethylene glycol 15-hydroxystearate | | 1:45 |
| mass ratio of phospholipid to polyethylene glycol 15-hydroxystearate | | 1:16 |
| phosphate concentration | | 0.025 mol/L |

### Preparation method:

1) polyethylene glycol 15-hydroxystearate, soybean lecithin S100 and oil were added into container 1, heated to 60 °C - 80 °C, and stirred to mix well; and cyclosporine was added to dissolve with stirring to form an oil phase;
2) about 4.5% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 25% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Example 6

Cyclosporine nanoemulsion was prepared using polyethylene glycol 15-hydroxystearate and soybean lecithin S100 as stabilizers, disodium hydrogen phosphate and sodium dihydrogen phosphate as pH regulators, and sodium chloride as an osmotic pressure regulator. The formulation composition is as follows:

| **Composition** | **Function** | **Amount** |
|---|---|---|
| cyclosporine | active substance | 0.1 g |
| oil (medium chain triglycerides) | dispersed phase | 1.5 g |
| polyethylene glycol 15-hydroxystearate | stabilizer | 3.0 g |
| soybean lecithin S100 | stabilizer | 0.218 g |
| sodium chloride | osmotic pressure regulator | 0.406 g |
| disodium hydrogen phosphate (anhydrous basis) | pH regulator | 0.511 g |
| sodium dihydrogen phosphate (anhydrous basis) | pH regulator | 0.168 g |
| water for injection | dispersing agent | up to 100ml |

| **Mass ratio/concentration** | | |
|---|---|---|
| mass ratio of cyclosporine to polyethylene glycol 15-hydroxystearate | | 1:30 |
| mass ratio of phospholipid to polyethylene glycol 15-hydroxystearate | | 1:14 |
| phosphate concentration | | 0.05 mol/L |

### Preparation method:

1) polyethylene glycol 15-hydroxystearate, soybean lecithin S100 and oil were added into container 1, heated to 60 °C - 80 °C, and stirred to mix well; and cyclosporine was added to dissolve with stirring to form an oil phase;
2) about 9% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 50% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Example 7

Cyclosporine nanoemulsion was prepared using polyethylene glycol 15-hydroxystearate and soybean lecithin S100 as stabilizers, disodium hydrogen phosphate and sodium dihydrogen phosphate as pH regulators, and sodium chloride as an osmotic pressure regulator. The formulation composition is as follows:

| **Composition** | **Function** | **Amount** |
|---|---|---|
| cyclosporine | active substance | 0.1 g |
| oil (medium chain triglycerides) | dispersed phase | 1.5 g |
| polyethylene glycol 15-hydroxystearate | stabilizer | 4.5 g |
| soybean lecithin S100 | stabilizer | 0.146 g |
| sodium chloride | osmotic pressure regulator | 0.406 g |
| disodium hydrogen phosphate (anhydrous basis) | pH regulator | 0.511 g |
| sodium dihydrogen phosphate (anhydrous basis) | pH regulator | 0.168 g |
| water for injection | dispersing agent | up to 100ml |

| **Mass ratio/concentration** | | |
|---|---|---|
| mass ratio of cyclosporine to polyethylene glycol 15-hydroxystearate | | 1:45 |
| mass ratio of phospholipid to polyethylene glycol 15-hydroxystearate | | 1:31 |
| phosphate concentration | | 0.05 mol/L |

### Preparation method:

1) polyethylene glycol 15-hydroxystearate, soybean lecithin S100 and oil were added into container 1, heated to 60 °C - 80 °C, and stirred to mix well; and cyclosporine was added to dissolve with stirring to form an oil phase;
2) about 9% of the total amount of water for injection was added into container 2, an appropriate amount of osmotic pressure regulator was added under stirring, and stirring continued until dissolution to form a primary emulsion aqueous phase;
3) under stirring, the primary emulsion aqueous phase was added into the oil phase, gradually warmed to 60 °C - 80 °C under stirring, and stirring continued for 5-15 minutes to form a primary emulsion;
4) about 50% of the total amount of water for injection was added into container 3, the pH regulator and the remaining osmotic pressure regulator were added under stirring, and stirring continued until dissolution to form a diluted aqueous phase;
5) under stirring, the primary emulsion was added to the diluted aqueous phase, container 2 rinsed several times with appropriate amount of water for injection and the rinse water transferred to container 3, and stirring was continued for 5-10 minutes;
6) water for injection was added to the total amount of the formulation and stirred well.

### Experimental Example 8: Investigation of the Quality Characteristics of Cyclosporine Nanoemulsions

In this application, the particle size is determined according to the third method of General Principle 0982 in Part 4 of the 2020 edition of the Chinese Pharmacopoeia; and the encapsulation rate is tested as follows:
Take 200 µl of this product and add it to the top of the dextran gel G-25 microcolumn, centrifuge it at 2000 rpm/min for 1 min, elute it with water by adding 200 µl of water to the top of the microcolumn and centrifuging it at 2000 rpm/min for 2 min, with the elution process repeated 4 times, collect the eluate in a 5 ml volumetric bottle, add methanol to break the emulsion and dilute to the scale, and shake well. Take an appropriate amount of cyclosporine reference substance, add 500 µl of methanol to dissolve it, and then quantitatively dilute with 50% methanol solution to make a solution containing about 0.1 mg of cyclosporine reference substance per 1 ml. Use octadecylsilane bonded silica gel as filler; acetonitrile-water (70:30) as mobile phase; flow rate of 1.0 ml per minute; stainless steel tube and column temperature of 80°C; detection wavelength of 210 nm. Take the above solutions respectively, inject them into the liquid chromatograph, record the chromatograms, and make calculation based on the peak areas according to the external standard method.

Samples were prepared according to the formulation and process of Example 1, and the properties, pH value, particle size and encapsulation rate of the cyclosporine nanoemulsion were investigated. The results are shown in Table 1. Samples were prepared according to the formulations and processes of Examples 2, 3, 4, 5, 6, and 7, and the properties, pH value and particle size of the cyclosporine nanoemulsions were investigated. The results are shown in Table 2.

**Table 1. Quality characteristics of the cyclosporine nanoemulsion of Example 1**

| Item | Characteristics | pH | Particle size | | Encapsulation rate |
|---|---|---|---|---|---|
| | | | Average particle size | D₉₀ | |
| Limit requirements | Should be an almost transparent milky liquid | 6.5-8.0 | ≤50 nm | ≤90 nm | ≥90% |
| Example 1 | Almost transparent milky liquid | 7.2 | 23 nm | < 27 nm | 98.7% |

**Table 2. Quality characteristics of the cyclosporine nanoemulsions of Examples 2-6**

| Item | Characteristics | pH | Particle size | |
|---|---|---|---|---|
| | | | Average particle size | D₉₀ |
| Limit requirements | Should be an almost transparent milky liquid | 6.5-8.0 | ≤50 nm | ≤90 nm |
| Example 2 | Almost transparent milky liquid | 7.2 | 22 nm | < 23 nm |
| Example 3 | Almost transparent milky liquid | 7.3 | 22 nm | < 25 nm |
| Example 4 | Almost transparent milky liquid | 7.2 | 26 nm | < 43 nm |
| Example 5 | Almost transparent milky liquid | 7.2 | 24 nm | < 30 nm |
| Example 6 | Almost transparent milky liquid | 7.3 | 26 nm | < 31 nm |
| Example 7 | Almost transparent milky liquid | 7.2 | 22 nm | < 26 nm |

Conclusion: The properties, pH value, particle size and encapsulation rate of the cyclosporine nanoemulsions meet the limit requirements.

### Experimental Example 9: Stability Tests of the Cyclosporine Nanoemulsions

Samples were prepared according to the formulations and processes of Examples 1, 2 and 3, and the high temperature stability at 40 °C and light exposure stability at 5000 ± 500 lx were investigated.

The results are shown in Tables 3 and 4. Samples were prepared according to the formulation and process of Example 1, and the intermediate condition test at 30 °C and the long-term test at 15-20 °C were performed. The results are shown in Tables 5 and 6.

**Table 3. High temperature stability test results of cyclosporine nanoemulsions of Examples 1 to 3**

| Item | | Characteristics | pH | Particle size | |
|---|---|---|---|---|---|
| | | | | Average particle size | D₉₀ |
| Limit requirements Sample (day) | | Should be an almost transparent milky liquid | 6.5-8.0 | ≤50 nm | ≤90 nm |
| Example 1 | 0 | Almost transparent milky liquid | 7.2 | 23 nm | < 27 nm |
| | 30 | Almost transparent milky liquid | 7.1 | 23 nm | < 31 nm |
| Example 2 | 0 | Almost transparent milky liquid | 7.2 | 22 nm | < 23 nm |
| | 30 | Almost transparent milky liquid | 7.1 | 20 nm | < 21 nm |
| Example 3 | 0 | Almost transparent milky liquid | 7.3 | 22 nm | < 25 nm |
| | 30 | Almost transparent milky liquid | 7.2 | 24 nm | < 32 nm |

**Table 4. Light exposure stability test results of cyclosporine nanoemulsions of Examples 1 and 3**

| Item | | Characteristics | pH | Particle size | |
|---|---|---|---|---|---|
| | | | | Average particle size | D₉₀ |
| Limit requirements Sample (day) | | Should be an almost transparent milky liquid | 6.5-8.0 | ≤50 nm | ≤90 nm |
| Example 1 | 0 | Almost transparent milky liquid | 7.2 | 23 nm | < 27 nm |
| | 30 | Almost transparent milky liquid | 7.1 | 24 nm | < 33 nm |
| Example 3 | 0 | Almost transparent milky liquid | 7.3 | 22 nm | < 25 nm |
| | 30 | Almost transparent milky liquid | 7.2 | 22 nm | < 27 nm |

**Table 5. Intermediate Condition Stability Test Results of the Cyclosporine Nanoemulsion of Example 1**

| Item | Characteristics | pH | Particle size | | Encapsulation rate | |
|---|---|---|---|---|---|---|
| | | | Average particle size | D₉₀ | | |
| Limit requirements | Should be an almost transparent milky | 6.5-8.0 | ≤50 nm | ≤90 nm | ≥90% | |
| Sample (month) | | liquid | | | | |
| Example 1 | 0 | Almost transparent milky liquid | 7.2 | 23 nm | < 27 nm | 98.7% |
| | 3 | Almost transparent milky liquid | 7.2 | 25 nm | < 35 nm | 101.0% |
| | 6 | Almost transparent milky liquid | 7.1 | 25 nm | < 37 nm | - |

**Table 6. Long-Term Stability Test Results of the Cyclosporine Nanoemulsion of Example 1**

| Item | | Characteristics | pH | Particle size | | Encapsulation rate |
|---|---|---|---|---|---|---|
| | | | | Average particle size | D₉₀ | |
| Limit requirements Sample (month) | | Should be an almost transparent milky liquid | 6.5-8.0 | ≤50 nm | ≤90 nm | ≥90% |
| Example 1 | 0 | Almost transparent milky liquid | 7.2 | 23 nm | < 27 nm | 98.7% |
| | 3 | Almost transparent milky liquid | 7.2 | 23 nm | < 30 nm | 97.7% |
| | 6 | Almost transparent milky liquid | 7.2 | 24 nm | < 34 nm | - |

Conclusion: The pH value, particle size and encapsulation rate of the cyclosporine nanoemulsions are of good stability, indicating that the use of buffer salt can improve the pH stability of the emulsion, and the interfacial composite film composed of polyethylene glycol 15-hydroxystearate and phospholipid can tolerate higher ionic strength and ensure the stability of the emulsion particles and encapsulation during the storage of the nanoemulsion.

### Experimental Example 10: Pharmacodynamics of cyclosporine nanoemulsion on dry eye in mice induced by dryness stress

### 1. Test drug:

Low-dose test sample: 0.05% cyclosporine nanoemulsion, prepared according to the formulation and process of Example 5.

Medium-dose test sample: 0.1% cyclosporine nanoemulsion, prepared according to the formulation and process of Example 1.

High-dose test sample: 0.3% cyclosporine nanoemulsion, prepared according to the formulation and process of Example 4.

Commercially available reference: Ikervis^{®} ; specification: 0.1%.

Blank excipient: prepared according to the formulation and process of Example 1, except that cyclosporine was not included.
2. Test animals: C57BL/6JShjh mice, weighing 15.4-20.4 g, female.
3. Experimental methods: from 150 healthy female C57BL/6JShjh mice, on D-1, 21 mice with no abnormalities in both eyes were selected without modeling, and 125 mice subjected to low humidity environment combined with subcutaneous injection of scopolamine (twice/day, 0.75 mg/mL, 0.3 mL/mouse each time) for 5 consecutive days to induce modeling. The first day of modeling was recorded as D1. On D5, 15 non-modeling animals with similar corneal fluorescein sodium staining scores and tear secretion were selected and grouped into the non-modeling group. 90 modeling animals with similar corneal fluorescein sodium staining scores and tear secretion were selected with consideration of the basic scores before modeling, and randomly grouped into Groups 2-7 (15/group) according to the mean corneal fluorescein sodium staining scores of both eyes, i.e. the model control group, blank excipient group, commercial control group, and low, medium, and high dose groups of the test product in sequence. From D6 to D15, animals in groups 2 to 7 continued to be subjected to modeling (same method as before), groups 1 to 2 were not administered anything, and groups 3 to 7 were administered blank excipients, Ikervis, 0.05%, 0.1% and 0.3% cyclosporine nanoemulsion, respectively, by eye drops in both eyes (3 times/day, with an interval of about 3 hours, 3 µL/eye). Tear secretion and corneal fluorescein sodium staining tests were performed on animals in each group on D-1, D5, D10 and D15.
4. Evaluation method:
   Tear secretion: restrain the mouse, use clean toothless forceps to hold the shortened commercially available phenol red cotton thread, fix the cotton thread in the middle of the mouse lower eyelid conjunctival sac for 30 seconds, and measure the wetted length of the cotton thread. The tear secretion summary is shown in Table 7.

Corneal fluorescein sodium staining test: After dripping fluorescein sodium solution (1.5 µL, 0.5%) into the upper conjunctival sac of the animal, about 1.5 min after staining, use 1.25 mL of sterile saline to rinse the animal's conjunctival sac about every 10 s for 3 times. After each rinse, use tissue paper to absorb the saline around the animal's eyes. About 5 minutes after staining, use a slit lamp (+cobalt blue filter) to observe the ocular surface, take pictures, and score. Scoring criteria: Each cornea is divided into 5 areas (1-central area, 2-superior area, 3-temporal area, 4-nasal area, 5-inferior area). The maximum staining score for each area is 8 points, where 0 means that the corresponding area is not stained, 1 means that the area of punctate staining is 1%-25% of the area of the corresponding area, 2 means that the area of punctate staining is 26%-50% of the area of the corresponding area, 3 means that the area of punctate staining is 51%-75% of the area of the corresponding area, and 4 means that the area of punctate staining is 76%-100% of the area of the corresponding area. If the staining area is dense and/or obvious fusion is visible, 1, 2, 3, and 4 points will be added according to the staining area percentage relative to the corresponding area, that is, 1%~25%, 26%~50%, 51%~75%, and 76%~100%, respectively. The maximum total score for each eye is 40 points. The total score of corneal fluorescein sodium staining for each eye was calculated. The summary of corneal fluorescein sodium staining scores is shown in Table 8.

**Table 7. Summary of tear secretion (mm)**

| Group | | D-1 | D5 | D10 | D15 |
|---|---|---|---|---|---|
| 1-Non-modeling group | Mean±SD N | 5.48±1.89 30 | 5.77±2.12 30 | 6.22±2.07 30 | 6.65±2.21 30 |
| 2-Model control group | Mean±SD N | 5.93±2.20 30 | 1.50± 0.57^{a} 30 | 1.90±0.62^{a} 30 | 1.82±0.51^{a} 28 |
| 3-Blank excipient group | Mean±SD N | 5.97±1.96 30 | 1.43±0.61^{a} 30 | 1.82±0.50^{a} 30 | 1.82±0.70^{a} 30 |
| 4-Commercial control group | Mean±SD N | 6.02±2.13 30 | 1.55±0.63^{a} 30 | 3.00±0.71^{abc} 30 | 3.08±1.02^{abc} 30 |
| 5-Low dose group of test product | Mean±SD N | 5.28±1.88 30 | 1.48±0.59^{a} 30 | 2.78±0.99^{abc} 30 | 3.22±0.72^{abc} 30 |
| 6-Medium dose group of test product | Mean±SD N | 5.30±2.00 30 | 1.48±0.79^{a} 30 | 2.82±0.92^{abc} 30 | 3.37±0.54^{abc} 30 |
| 7-High dose group of test product | Mean±SD N | 5.37±1.91 30 | 1.62±0.54^{a} 30 | 2.72±0.67^{abc} 30 | 3.45±0.79^{abc} 30 |

| | | | | | |
|---|---|---|---|---|---|
| Note: N indicates the number of animal eyes. Compared with the non-modeling group, ^{a} indicates p≤0.05; compared with the model control group, ^{b} indicates p≤0.05; compared with the blank excipient group, ^{c} indicates p≤0.05; compared with the commercial control group, ^{d} indicates p≤0.05; compared with the low-dose group of the test product, ^{e} indicates p≤0.05; compared with the medium-dose group of the test product, ^{f} indicates p≤0.05. | | | | | |

**Table 8. Summary of corneal fluorescein sodium staining scores**

| Group | | D-1 | D5 | D10 | D15 |
|---|---|---|---|---|---|
| 1-Non-modeling group | Mean±SD N | 14.3±2.8 30 | 16.0±4.0 30 | 17.2±4.4 30 | 16.6±3.5 30 |
| 2-Model control group | Mean±SD N | 15.7±4.6 30 | 35.1±2.5^{a} 30 | 35.0±2.2^{a} 30 | 35.2±2.5^{a} 28 |
| 3-Blank excipient group | Mean±SD N | 15.6±3.2 30 | 35.4±2.0^{a} 30 | 32.1±3.8^{ab} 30 | 29.6±4.2^{ab} 30 |
| 4-Commercial control group | Mean±SD N | 15.3±4.3 30 | 35.2±2.0^{a} 30 | 27.7±4.5^{abc} 30 | 22.5±4.0^{abc} 30 |
| 5-Low dose group of test product | Mean±SD N | 15.2±3.7 30 | 35.8±1.8^{a} 30 | 24.7±4.0^{abcd} 30 | 21.8±4.5^{abc} 30 |
| 6-Medium dose group of test product | Mean±SD N | 14.3±3.8 30 | 34.9±2.2^{a} 30 | 21.7±3.7^{abcde} 30 | 19.5±3.3^{abcd} 30 |
| 7-High dose group of test product | Mean±SD N | 16.2±4.5 30 | 35.5±2.4^{a} 30 | 20.1±2.6^{abcde} 30 | 17.5±3.2^{bcdef} 30 |

| | | | | | |
|---|---|---|---|---|---|
| Note: N indicates the number of animal eyes. Compared with the non-modeling group, ^{a} indicates p≤0.05; compared with the model control group, ^{b} indicates p≤0.05; compared with the blank excipient group, ^{c} indicates p≤0.05; compared with the commercial control group, ^{d} indicates p≤0.05; compared with the low-dose group of the test product, ^{e} indicates p≤0.05; compared with the medium-dose group of the test product, ^{f} indicates p≤0.05. | | | | | |

### 5. Experimental results

A low humidity environment combined with subcutaneous injection of scopolamine could successfully induce a decrease in tear secretion in mice. Administration of low, medium and high doses of the test products and the commercially available control product for 5 consecutive days significantly improved tear secretion in model mice. When the administration was extended to 10 days, the low, medium and high doses of the test products and the commercially available control product still showed a certain improvement effect, and the effect of the test products was more obvious in terms of the mean.

A low humidity environment combined with subcutaneous injection of scopolamine could successfully induce an increase in the corneal fluorescein sodium staining score of mice. Administration of low, medium and high doses of the test products and the commercially available control product for 5 consecutive days could significantly reduce the corneal fluorescein sodium staining score of mice. When the administration was extended to 10 days, the corneal fluorescein sodium staining score of mice could still be reduced. The effects of medium and high doses of test products were better than those of the commercially available control product, and the high dose of test products could restore the corneal fluorescein sodium staining score of model mice to close to normal levels.

Conclusion: The cyclosporine ophthalmic emulsion of the present invention can improve the drug efficacy.

Although certain features of the invention have been illustrated and described herein, many modifications, substitutions, changes and equivalents will occur to those skilled in the art. It should be understood, therefore, that the appended claims are intended to cover all such modifications and changes that fall within the true spirit of the invention.

## Claims

1. An oil-in-water cyclosporine emulsion, preferably a nanoemulsion, comprising an interfacial stabilizer, wherein the interfacial stabilizer comprises a combination of polyethylene glycol 15-hydroxystearate and a phospholipid.

2. The oil-in-water cyclosporine emulsion according to claim 1, further comprising a buffer salt.

3. The oil-in-water cyclosporine emulsion according to claim 1, wherein the phospholipid is selected from the group consisting of soybean lecithin, egg yolk lecithin, and any combination thereof, preferably, selected from the group consisting of soybean lecithin S100, egg yolk lecithin E80, and any combination thereof.

4. The oil-in-water cyclosporine emulsion according to claim 1, wherein the mass ratio of cyclosporine to polyethylene glycol 15-hydroxystearate is 1:30 to 1:45.

5. The oil-in-water cyclosporine emulsion according to claim 1, wherein the mass ratio of phospholipid to polyethylene glycol 15-hydroxystearate is 1:14 to 1:31.

6. The oil-in-water cyclosporine emulsion according to claim 1, wherein the content of polyethylene glycol 15-hydroxystearate is 2.25-13.5 g/100 ml.

7. The oil-in-water cyclosporine emulsion according to claim 2, wherein the buffer salt is a phosphate buffer salt, and preferably, the buffer salt concentration is 0.01 mol/L to 0.05 mol/L calculated on the basis of phosphate.

8. The oil-in-water cyclosporine emulsion according to claim 1, further comprising an osmotic pressure regulator; preferably, the osmotic pressure regulator is selected from the group consisting of sodium chloride, sorbitol, and any combination thereof; preferably, the content of the osmotic pressure regulator is 0.2-1 g/100 ml.

9. The oil-in-water cyclosporine emulsion according to claim 1, wherein the average particle size of the particles of the emulsion does not exceed 50 nm, and preferably is 10-40 nm, more preferably 20-30 nm; preferably, 90% of the particles of the emulsion have a particle size of no more than 90 nm; preferably, the pH value of the emulsion is 6.5-8.0, preferably 6.8-7.6, more preferably 7.0-7.4; preferably, the encapsulation rate of the emulsion is not less than 90%, preferably not less than 95%; preferably, the emulsion is a transparent milky liquid; preferably, the emulsion is an eye drop; preferably, the oil is selected from the group consisting of medium-chain triglycerides, soybean oil, castor oil, olive oil, and fish oil.

10. A method for preparing an oil-in-water cyclosporine emulsion, wherein the emulsion comprises an interfacial stabilizer, an osmotic pressure regulator and a buffer salt, wherein the interfacial stabilizer comprises a combination of polyethylene glycol 15-hydroxystearate and a phospholipid, and the method comprises
1) mixing the oil and the interfacial stabilizer uniformly, adding cyclosporine thereto and dissolving the cyclosporine to form an oil phase;
2) dissolving a portion of the osmotic pressure regulator in water, and stirring and mixing the oil phase and the water containing the osmotic pressure regulator to form a primary emulsion; and
3) dissolving the buffer salt and the remaining osmotic pressure regulator in water to form a solution, and adding the primary emulsion to the solution and mixing well.
